# EUROPEAN PATENT APPLICATION

(11) **EP 4 685 117 A1**
(43) Date of publication of application: **28.01.2026**
(21) Application number: 24201427.2
(22) Date of filing: 19.09.2024
(51) Int. Cl.: C02F 11/02, C02F 11/10, C02F 11/12, C05F 7/00

(54) **SEWAGE SLUDGE-TO-RESOURCE RECYCLING SYSTEM OF CIRCULATION-TYPE**

(30) Priority: 24.07.2024 JP 2024118329
(71) Applicant: Moritomizu Giken Co., Ltd., Adachi-ku, Tokyo (JP)
(72) Inventor: TAMAKI, Masatomo, Chiba (JP); OHYAMA, Mitsutoshi, Tokyo (JP)
(74) Representative: Jacobacci Coralis Harle

(57) **Abstract**

The sewage sludge-to-resource recycling system of circulation-type according to the present invention comprises: dehydration means to dehydrate sewage sludge to form a raw material; primary fermentative treatment means comprising a horizontal cylindrical shaped fermentative treatment container configured to be rotated about a central axis thereof; raw material supply means configured to receive the raw material from the dehydration means and supply the received raw material into the fermentative treatment container; primary fermented product discharge means configured to discharge the primary fermented product outside the fermentative treatment container: secondary fermentative treatment means to receive the primary fermented product from the primary fermentative treatment means, and additionally ferment the received primary fermented product to produce a secondary fermented product having a second set water content value or less, the second set water content value being less than a water content of the primary fermented product; carbonization means to receive the secondary fermented product from the secondary fermentative treatment means, and subject the received secondary fermented product to carbonization treatment to produce a carbide; mixing means to receive the secondary fermented product from the secondary fermentative treatment means and the carbide from the carbonization means, and mix them together to produce a mixture; wherein the mixing means is connected to third distribute and supply means, the third distribution and supply means being configured to distributively supply at least part of the mixture to the raw material supply means and/or third useful resource processing means to perform processing into a third useful product of a third form.

## Description

### TECHNICAL FIELD

The present invention relates to a sewage sludge-to-resource recycling system of circulation-type.

### BACKGROUND ART

The Ministry of Land, Infrastructure, Transport and Tourism (MLIT) in Japan issued the following notice to the directors of the sewage departments in each prefecture and each ordinance-designated city, by the circular notice KMGK No. 99 dated March 17, 2023.

### " BASIC POLICY ON TREATMENT OF GENERATED SLUDGE OR THE LIKE

The Sewerage Service Act Article 21bis (2) stipulates that "in treating generated sludge or the like, efforts shall be made to reduce the amount thereof by dehydration, incineration, etc., and to enable generated sludge to be reusable as fuel or fertilizer". Further, in Japan, the realization of carbon neutrality by 2050 and the conversion of production materials to domestic alternatives toward strengthening food security are important issues. Under these circumstances, the need for utilizing sewage sludge as energy and fertilizer is growing even more.

In particular, the "Outline of Food Security Strengthening Policy" (decided by the Headquarters for Stable Food Supply and Strengthening Infrastructure of Agricultural, Forestry and Fishery Industries, on December 27, 2022) states that the utilization amount of sewage sludge-based resources and composted fertilizer will be doubled, and the percentage of domestic resources in the usage of fertilizer (on a phosphorus basis) will be increased to 40%, by 2030

In light of this background, and in order to further expand contribution to the realization of a circulation-type society through sewage projects, a basic policy regarding future treatments of generated sludge or the like has been established as follows. We ask that you fully understand this policy and endeavor to implement sewage projects.

We would like to ask each prefectural government to inform the municipalities in your jurisdiction (excluding ordinance-designated cities) to this effect.

Please note that this notice is technical advice based on the Local Autonomy Act (Act No. 67, 1947) Article 245quater (1)."

As an apparatus for converting wastes such as sewage sludge into a resource, i.e., recycling organic wastes such as sewage sludge, there has been known a sewage sludge recycling system comprising:
a fermentative treatment container formed in a horizontal cylindrical shape and configured to fermentatively treat organic waste as a raw material by aerobic hyperthermophiles (hereinafter referred to occasionally as "super thermophiles");
raw material supply means comprising a raw material hopper which is provided at one end of the fermentative treatment container and configured to temporarily store organic waste as a raw material, the raw material supply means being configured to supply the raw material temporarily stored in the raw material hopper, into the fermentative treatment container; and
discharge means provided at the other end of the fermentative treatment container and configured to discharge a fermentatively-treated, or treated, raw material as the raw material subjected to the fermentative treatment, outside the fermentative treatment container,
wherein the fermentative treatment is performed while the fermentative treatment container is rotated about a central axis thereof to cause the raw material to gradually move within the fermentative treatment container from the one end side toward the other end side of the fermentative treatment container over a given number of days, thereby recycling the organic wastes, or converting the organic wastes into compost and fertilizer, i.e., a closed sewage sludge recycling system (hereinafter referred to as "closed composting apparatus").

With a view to providing a technique of generating compost from organic waste, and circulating the compost to generate new liquid fertilizer and/or compost, wherein the technique attains excellent energy efficiency and contributes to environmental conservation, JP2005-320182A proposes "an organic waste composting treatment system for recycling organic waste consisting of solid waste and liquid waste into compost and liquid fertilizer" in order to achieve this object. More specifically, the JP2005-320182A provides "an organic waste composting treatment system characterized in that it comprises:
a crushing and mixing unit comprising a crushing mechanism for crushing solid waste, and a microorganism injection mechanism for injecting and mixing a microorganism into and with the solid waste which has been crushed or is being crushed;
a decomposition and fermentation unit for delivering the solid waste subjected to the crushing and mixing by the crushing and mixing unit into a decomposition and fermentation mechanism for decomposing and fermenting the delivered soil waste by the activity of the microorganism, thereby generating ripe compost from the solid waste; and
a liquid fertilizer production unit for generating liquid using, as raw materials, a part of the ripe compost generated by the decomposition and fermentation unit and the liquid waste." as recited in claim 1 of the Patent Document 1. Further, in claim 2, the organic waste composting treatment system recited in claim 1 is specified such that the crushing and mixing unit comprises a circulation mechanism for injecting and mixing the ripe compost into and with the crashed solid waste.

The JP2005-320182A describes as follows: "According to the organic waste composting treatment system recited in the above claim 2, the circulation mechanism of the crushing and mixing unit introduces a part of the ripe compost into the unit. The crushing and mixing unit mixes the solid waste and the ripe compost together. Thus, since returning of the ripe compost is performed by the circulation mechanism, decomposition and fermentation can be promoted without the need for a microorganism injection mechanism such as a material for culture. This makes it possible to reduce the usage rate of a microorganism used in the microorganism injection mechanism, thereby contributing to a reduction in cost."

### CITATION LIST

### [Patent Document]

Patent Document 1: JP 2005-320182 A

### SUMMARY OF INVENTION

### [Technical Problem]

As described above, according to the organic waste composting treatment system recited in the above claim 2, since returning of the ripe compost is performed by the circulation mechanism, it is expected to obtain an advantageous effect of being able to promote decomposition and fermentation without the need for a microorganism extraction mechanism such as a material for culture, and reduce the usage rate of a microorganism used in the microorganism extraction mechanism, thereby contributing to a reduction in cost. On the other hand, however, it takes 30 to 90 days by the time the organic waste becomes usable ripe compost, as described in the Description of the Patent Document 1.

In contrast, the first-mentioned closed composting apparatus can produce ripe compost in about 20 days. However, depending on the conditions of a raw material, etc., the raw material can be discharged from the apparatus in an unripe state, in some cases.

It is therefore an object of the present invention to provide a sewage sludge-to-resource recycling system of circulation-type capable of solving the above-mentioned problem of the closed composting apparatus by: using, as a substructure, the structure of the closed composting system and further improving the closed composting system while taking advantage thereof, and incorporating the advantages of the organic waste composting treatment system of the JP2005-320182A.

### [Solution to Technical Problem]

The above technical problem is solved by a sewage sludge-to-resource recycling system of circulation-type having features set forth in the following sections (1) to (17).
(1) A sewage sludge-to-resource recycling system of circulation-type for recycling sewage sludge as organic waste into a useful product, the sewage sludge-to-resource recycling system of circulation-type comprising:
   dehydration means to dehydrate sewage sludge until a water content of the dehydrated sewage sludge becomes a preset given raw material water content value, to form a raw material;
   primary fermentative treatment means comprising a fermentative treatment container which is formed in a horizontal cylindrical shape and configured to be rotated about a central axis thereof, the primary fermentative treatment means being configured to rotate the fermentative treatment container about the central axis to cause the raw material within the fermentative treatment container to gradually move from an inlet end side toward a discharge end side of the fermentative treatment container over a given number of days, thereby fermentatively treating the raw material by an aerobic microorganism to produce a primary fermented product having a first set water content value or less;
   raw material supply means provided on the inlet end side of the fermentative treatment container and configured to receive the raw material from the dehydration means and supply the received raw material into the fermentative treatment container;
   primary fermented product discharge means provided on the discharge end side of the fermentative treatment container and configured to discharge the primary fermented product outside the fermentative treatment container:
      secondary fermentative treatment means to receive the primary fermented product from the primary fermentative treatment means, and additionally ferment the received primary fermented product to produce a secondary fermented product having a second set water content value or less, the second set water content value being less than a water content of the primary fermented product;
      carbonization means to receive the secondary fermented product from the secondary fermentative treatment means, and subject the received secondary fermented product to carbonization treatment to produce a carbide;
      mixing means to receive the secondary fermented product from the secondary fermentative treatment means and the carbide from the carbonization means, and mix them together to produce a mixture; and
      water content measurement means to measure the water content of the primary fermented product discharged from the primary fermentative treatment means;
      wherein:
         the primary fermented product discharge means is connected to first distribution and supply means, the first distribution and supply means being configured to, in a normal state in which a water content value of the primary fermented product measured by the water content measurement means is equal to or less than the first set water content value, distribute a first given amount of the primary fermented product and a remaining amount of the primary fermented product, respectively, to the secondary fermentative treatment means, and first useful resource processing means to perform processing into a first useful product of a first form, and in a non-normal state in which said water content value exceeds the first set water content value, supply an entire amount of the primary fermented product to the secondary fermentative treatment means;
         the secondary fermented product discharge means is connected to second distribution and supply means, the second distribution and supply means being configured to distributively supply at least part of the secondary fermented product to the carbonization means and the mixing means; and
         the mixing means is connected to third distribute and supply means, the third distribution and supply means being configured to distributively supply at least part of the mixture to the raw material supply means and/or third useful resource processing means to perform processing into a third useful product of a third form.
(2) The sewage sludge-to-resource recycling system set forth in the section (1), wherein the secondary fermentative treatment means is one selected from the group consisting of: heaping-type fermentative treatment means using a compost depot; an open-type fermentative treatment means using a rotary scoop for agitation; and a closed agitation-type fermentative treatment means using a composting tank.
(3) The sewage sludge-to-resource recycling system set forth in the section (2), wherein
   the aerobic microorganism is gram-positive aerobic thermophiles, and
   the secondary fermentative treatment means is the heaping-type fermentative treatment means, and comprises: a production part to heap the primary fermented product supplied from the first distribution and supply means, and fermentatively treat the heaped primary fermented product by the gram-positive aerobic thermophiles to produce the secondary fermented product such that it has the second set water content value less than the first set water content value for the primary fermented product; a storage part to store the secondary fermented product produced in the production part; and secondary fermented product discharge means to discharge the secondary fermented product from the storage part by a given amount.
(4) The sewage sludge-to-resource recycling system set forth in the section (1), wherein the third distribute and supply means is configured to, in the non-normal state, distributively supply the mixture to the raw material supply means.
(5) The sewage sludge-to-resource recycling system set forth in the section (1), wherein the third distribute and supply means is configured to supply a given amount of the mixture to a biological treatment part of a sewage treatment facility via microorganism activation means, in a state in which the aerobic microorganism in the secondary fermented product of the mixture is activated.
(6) The sewage sludge-to-resource recycling system set forth in the section (3), wherein the secondary fermentative treatment means comprises a supply conveyer for transferring the primary fermented product supplied from the first distribution and supply means, to the production part.
(7) The sewage sludge-to-resource recycling system set forth in the section (3), wherein the secondary fermentative treatment means comprises transfer means to transfer the secondary fermented product produced in the production part, from the production part to the storage part.
(8) The sewage sludge-to-resource recycling system set forth in the section (3), wherein the production part of the secondary fermentative treatment means comprises:
   second water content measurement means to measure a water content value of the produced secondary fermented product;
   feeding and returning means provided within the production part over an approximately entire length of the production part and configured to be switchable between a feeding state in which the primary fermented product is fed from an upstream side to a downstream side of the production part, and a return state in which the secondary fermented product is returned from the downstream side to the upstream side; and
   operational control means to control an operational state of the feeding and returning means such that it is switched between the feeding state and the return state, depending on the water content value of the secondary fermented product measured by the second water content measurement means.
(9) The sewage sludge-to-resource recycling system set forth in the section (8), wherein the operational control means is configured to control the operational state of the feeding and returning means such that it is switched to the return state when the water content value of the secondary fermented product measured by the second water content measurement means exceeds the second set water content value.
(10) The sewage sludge-to-resource recycling system set forth in the section (1), wherein the first set water content value is a value of 35% or less, and the second set water content value is set to be less than the first set water content value by 2 to 15%.
(11) The sewage sludge-to-resource recycling system set forth in the section (1), wherein the mixture after being processed by the third useful resource processing means is a material usable as both ameliorant and compost/fertilizer.
(12) The sewage sludge-to-resource recycling system set forth in the section (1), wherein the fermentative treatment container is provided with agitation means to agitate the raw material thereinside.
(13) The sewage sludge-to-resource recycling system set forth in the section (12), wherein the agitation means is rotation actuation means to rotate the fermentative treatment container.
(14) The sewage sludge-to-resource recycling system set forth in the section (1), wherein the raw material supply means comprises:
   a raw material hopper configured to temporarily store the raw material from the dehydration means;
   a jacket surrounding the raw material hopper with a gap relative to an outer wall of the raw material hopper, to form a heating gas space for allowing a heating gas to pass therethrough to heat the raw material within the raw material hopper; and
   a heating gas passage extending from the discharge end side of the fermentative treatment container to the heating gas space, the heating gas passage being configured to supply, as the heating gas, a discharge gas generated due to fermentation within the fermentative treatment container, from the fermentative treatment container to the heating gas space.
(15) The sewage sludge-to-resource recycling system set forth in the section (1), wherein the aerobic microorganism includes microorganisms belonging to gram-positive Bacilli of Firmicutes, and/or microorganisms belonging to gram-positive Actinobacteria of Actinomycetota, and/or gram-positive bacteria of Chloroflexi.
(16) The sewage sludge-to-resource recycling system set forth in any one of the sections (1) to (15), wherein the carbonization means is configured to use the secondary fermented product as a raw material to produce activated carbon.
(17) The sewage sludge-to-resource recycling system set forth in the section (1), wherein the second distribution and supply means is configured to distributively supply at least part of the secondary fermented product to the raw material supply means and/or second useful resource processing means to perform processing into a second useful product of a second form.

### [Advantageous Effects of Invention]

The sewage sludge-to-resource recycling system of circulation-type according to the present invention is configured to: produce a primary fermented product (typically, water content: 35% or less) by the primary fermentative treatment means; further ferment the primary fermented product to produce a secondary fermented product having a water content less than that of the primary fermented product; and carbonize the secondary fermented product to produce a carbide such as activated carbon. This carbide can be used as a water content adjuster to normalize fermentation of dehydrated sludge, or can be put into a biological treatment tank in a sewage treatment facility to achieve significant reduction in BOD (Biochemical Oxygen Demand) or COD (Chemical Oxygen Demand). In addition, the present invention is configured to produce a mixture of carbide and the secondary fermented product, so that the microorganism in the secondary fermented product also contribute to sewage treatment.

### BRIEF DESCRIPTION OF DRAWINGS

FIG. 1 is a diagram conceptually showing a sewage sludge-to-resource recycling system of circulation-type according to one embodiment of the present invention.
FIG. 2 is a detail diagram showing the details of a primary fermentative treatment device, etc., of the sewage sludge-to-resource recycling system of circulation-type illustrated in FIG. 1.

### DESCRIPTION OF EMBODIMENTS

With reference to the accompanying drawings, a sewage sludge-to-resource recycling system of circulation-type according to one embodiment of the present invention will now be described.

The sewage sludge-to-resource recycling system 10 comprises: a primary fermentative treatment device 11 comprising a fermentative treatment container 12 which is formed in a horizontal cylindrical shape and configured to fermentatively treat sewage sludge, i.e., organic waste, as a raw material by an aerobic microorganism, in particular, aerobic thermophiles; raw material supply means 20 provided at an inlet end 12a of the fermentative treatment container; and discharge means 30 provided at a discharge end 12b of the fermentative treatment container 12 to serve as a discharge gate for discharging a primary fermented product produced through the fermentative treatment of the raw material m, outside the fermentative treatment container. The sewage sludge-to-resource recycling system 10 is configured to control the raw material supply means 20 and the discharge means 30 by an operational synchronization means 140 such that they are operationally synchronized with each other.

The raw material supply means 20 comprises a raw material hopper 22 configured to temporarily store the raw material m from dehydration means 15 configured to dehydrate sewage sludge as organic waste until a water content of the dehydrated sewage sludge becomes a preset given raw material water content value, to form the raw material m. This raw material hopper 22 is provided with a conveyer 24 for supplying the raw material temporarily stored thereinside into the fermentative treatment container 12.

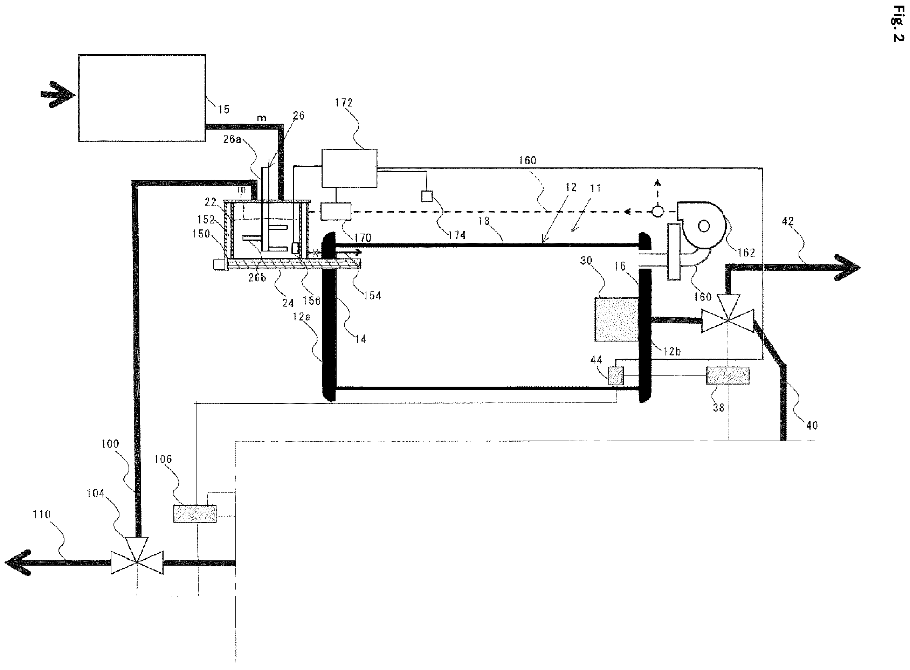

As shown in FIG. 2, the raw material hopper 22 is provided with rotating agitation means 26 which comprises a rotatory shaft 26a extending vertically within the raw material hopper and an agitation member 26b, such as horizontally-extending agitation blades, attached to the rotatory shaft 26a, wherein the agitating means 26 is configured to rotate at a circumferential velocity (e.g., about 0.1 to 0.5 m/s) that does not cause flying-up of the raw material and diffusion of odor. This agitation of the raw material makes it possible to achiever uniformize of water content distribution in the raw material.

As shown in FIG. 2, the fermentative treatment container 12 comprises a pair of fixed end plates 14, 16, and a cylindrical fermentative treatment drum 18 attached between the end plates rotatably about a central axis of the fermentative treatment container 12. An inner wall of the fermentative treatment drum 18 may be provided with forwarding blades (not illustrated). The fermentative treatment drum 18 is configured to be rotationally driven by a rotary drive unit (not illustrated) so as to cause the raw material put in the fermentative treatment container through the inlet end 12a to gradually move toward the discharge end 12b by the forwarding blades over a given number of days (e.g., about 21 days), thereby conducting fermentative treatment to recycle sewage sludge, i.e., convert sewage sludge into resources, such as comport and fertilizer. The above-mentioned movement of the raw material within the fermentative treatment container 12 may be based on pushing out of the raw material by the conveyor 24.

The fermentation is performed by an aerobic microorganism, in particular, aerobic thermophiles. According to the "Dictionary of Biochemistry (4th edition)" published by Tokyo Kagaku Doujin K.K., thermophiles are bacteria that can generally grow at 55°C or more, wherein thermophiles that can grow at 75°C or more, and thermophiles that can grow at 90°C or more, are referred to respectively, as "extreme thermophiles", and "hyperthermophiles". In the present invention, it is desirable to use bacteria (microorganism) that are extreme thermophiles or more thermophilic. For example, as aerobic extreme thermophiles, Thermus thermophiles may be used. For example, when a target resource is compost or fertilizer, plant seeds, pathogens, etc., in the material that are harmful to plant growth can be decomposed and killed by fermenting organic waste using such aerobic extreme thermophiles.

The sewage sludge-to-resource recycling system 10 comprises a secondary fermentative treatment facility 80 configured to receive the primary fermented product from the primary fermentative treatment device 11 and further ferments it (with drying) to produce a secondary fermented product having a lower water content value than the primary fermented product. The secondary fermented product is primarily intended to be used such that it functions as a part of a raw material water content-adjusting material for adjusting the water content value of the raw material, and is secondarily intended to provide a material for compost or fertilizer, having a different form (aspect) than the above. The secondary fermentative treatment facility 80 comprises a production part 82 disposed on a primary fermented product receiving side and configured to fermentatively treat the received primary fermented product to produce a secondary fermented product, and a storage part 84 configured to store the secondary fermented product produced in the production part 82. Details of the structure will be described below.

The discharge gate 30 is connected to a discharge passage 32 for the primary fermented product, and a first distribution and supply device 34 is provided at a downstream end of the discharge passage 32. This distribution and supply device 34 comprises a three-way valve 36, and operational control means 38 for controlling the three-way valve 36. The three-way valve 36 has a first discharge port and a second discharge, wherein the first discharge port is connected to a first supply passage 40 for supplying the primary fermented product in a first set amount to the secondary fermentative treatment facility 80 for processing the primary fermented product into the secondary fermented product, and the second discharge port is connected to a second supply passage 42 for supplying the primary fermented product in a second set amount to first useful resource processing means (not shown) to process the primary fermented product into a first useful product. The secondary fermentative treatment facility 80 comprises a supply conveyor, and the primary fermented product from the first supply passage 40 is supplied to the production part 82 of the secondary fermentative treatment facility 80 by the supply conveyor.

The operational control means 38 is connected to an operation panel 70 which is operated by an operator to issue instructions to the operational control means 38. The operator operates the operation panel 70 to adjust a distribution ratio (distribution amounts) of the primary fermented product to be distributed by the three-way valve 36 when the primary fermentative treatment device 11 normally functions, via the operational control means 38. For example, the distribution ratio (distribution amounts) may be set to 1/5 and 4/5, respectively, for the first supply passage 40 and the second supply passage 42.

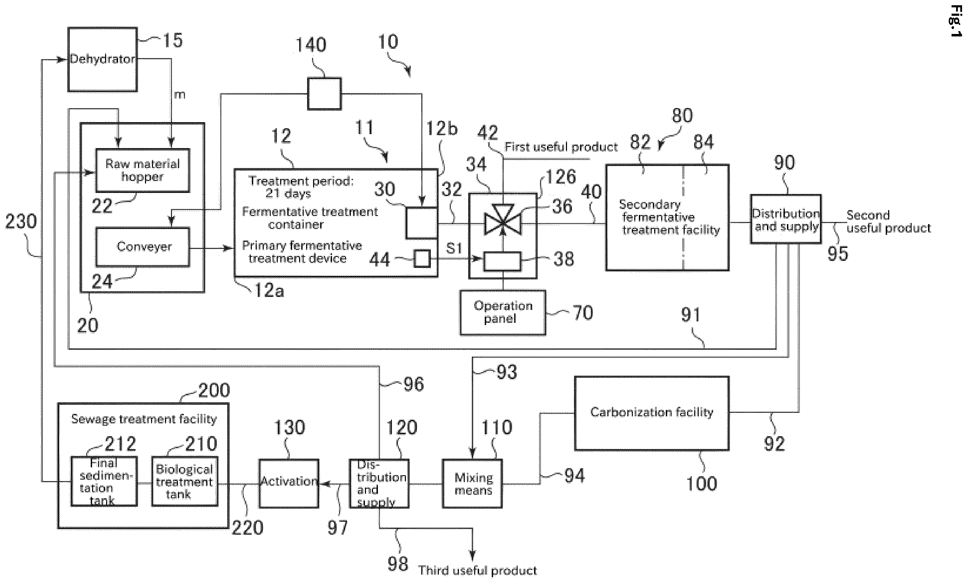

A water content sensor 44 is disposed in a primary fermented product discharge portion of the primary fermentative treatment device 11, e.g., at the discharge end 12b provided with the discharge gate 30 of the fermentative treatment container 12, as shown in FIG. 1. The water content sensor 44 is configured to measure the water content of the primary fermented product discharged from the fermentative treatment container 12, and output a signal S1 indicating a value of the measured water content, to the operational control means 38.

The operational control means 38 compares the received signal S1 indicating the water content value of the primary fermented product with a given water content value (e.g., 35% indicating a given ripeness of fertilizer) to determine whether the received signal S 1 is equal to or less than the given water content value (normal state) or is greater than the given water content value (non-normal state), wherein in the normal state, the operational control means 38 controls the three-way valve 36 to distribute the primary fermented product at the above ratio, and on the other hand, in the non-normal, controls the three-way valve 36 to close the second supply passage 42 and supply the entire amount of the primary fermented product to the first supply passage 40. In this way, the primary fermented product is not supplied to the first useful resource processing means when the primary fermented product is not in a desirable state for a first useful resource. Therefore, in the sewage sludge-to-resource recycling system according to this embodiment, no defective useful resource goes into the outside.

The production part 82 of the secondary fermentative treatment facility 80 serve as a secondary fermentative treatment means. This secondary fermentative treatment means may be any one selected from the group consisting of: heaping-type fermentative treatment means using a compost depot; an open-type fermentative treatment means using a rotary scoop for agitation; and a closed agitation-type fermentative treatment means using a composting tank, which have heretofore been used for production of compost.

The secondary fermentative treatment facility is preferably the heaping-type fermentative treatment means, wherein the primary fermented product heaped in the production part 82 is subjected to high-temperature aerobic fermentation, using an aerobic microorganism, in particular, gram-positive aerobic thermophiles, while performing turnover operations, under the condition where the temperature is maintained at 60°C to 110°C, to produce the secondary fermented product such that it has a second set water content value less than the first set water content value for the primary fermented product. This high-temperature aerobic fermentation decomposes and kills gram-negative anaerobic and aerobic microorganisms, but allows the aerobic microorganism used in the fermentation to stay alive as spores.

Preferably, the aerobic microorganism includes microorganisms belonging to gram-positive Bacilli of Firmicutes, and/or microorganisms belonging to gram-positive Actinobacteria of Actinomycetota, and/or gram-positive bacteria of Chloroflexi.

The production part 82 can be configured as follows.

A blower fan is provided on the primary fermented product-receiving side of the production part 82 to blow oxygen (air) into the primary fermented product heaped in the production part 82, and feeding and returning means composed of a conveyor or the like is provided over approximately the entire length of the inside of the production part 82 (from the primary fermented product-receiving side to the secondary fermented product-discharging side), wherein the primary fermented product supplied to the secondary fermentative treatment facility 80 is fermented while being fed by the feeding and returning means, and converted to the second fermented product such that it has a given second set water content value (i.e., a water content less than the water content of the primary fermented product; the second set water content value is set to be less than the first set water content value by 2 to 15%, particularly by 5%, i.e., in specific example, the second set water content value is preferably 30% when the first set water content value is 35%). Preferably, the feeding and returning means be provided with operational control means to perform control of switching between feeding and returning operations thereof, setting of feeding speed, etc.

The secondary fermentative treatment facility 80 comprises transfer means (not shown) provided on the discharge side of the production part 82 to transfer the produced secondary fermented product from the production part 82 to the storage part 84. The transfer means is configured such that the operation thereof is controlled by an operational control means.

A water content sensor is provided at a portion (downstream end) of the production part 82 of the secondary fermentative treatment facility 80 adjacent to the storage part 84. This water content sensor is provided as a means to monitor whether the primary fermented product which has been fed while being fermented to the downstream end of the production part 82 has the given water content of the desired secondary fermented product, and is configured to measure the water content of the primary fermented product at the downstream end of the production part 82 (secondary fermented product) and send a signal S2 indicating the measured water content value to the operational control means.

Upon receiving the signal S2, the operational control means operates to determine whether the water content value indicated by the signal is equal to or less than the given water content value (30%) for the secondary fermented product, and when the determination is YES, keep the feeding and returning means in the feeding state. On the other hand, when the determination is NO, the operational control means operates to switch the feeding and returning means to the returning state to return the primary fermented product to the upstream side and further perform fermentation. The above process will be repeated until the water content of the primary fermented product falls below the given water content for the secondary fermented product.

Further, upon receiving the signal S2, the operational control means operates to determine whether the water content value indicated by the signal is equal to or less than the given water content value (30%) for the secondary fermented product, and when the determination is YES, activate the transfer means to transfer the produced secondary fermented product to the storage part 84. On the other hand, when the determination is NO, the operational control means operates to avoid activating the transfer means, thereby preventing an incomplete secondary fermented product from being transferred to the storage part 84.

A second distribution and supply device 90 is connected to the discharge end side of the storage part 84. This second and distribution and supply device 90 may have approximately the same configuration as that of the first distribution and supply device 34, except that a valve mechanism functioning as a five-way valve is used, instead of the three-way valve, and thus detailed description of its structure, etc. is omitted. The second distribution and supply device 90 is configured to distributively supply at least part of the secondary fermented product to the raw material supply means 20, the after-mentioned carbonization facility 100, mixing means 110, and second useful resource processing means (not shown) for processing into a second useful product of a second form.

The second distribution and supply device 90 may be configured to supply the secondary fermented product having a water content as low as 30% or less, to the raw material supply means 20, during the non-normal state. In this case, the secondary fermented product is primarily used to function as a raw material water content-adjusting material for adjusting the water content of the raw material, but it also acts to add, to the raw material, aerobic thermophiles which are carried by its solid part and functions for fermentative treatment. For feeding of the secondary fermented product, the sewage sludge-to-resource recycling system 10 comprises a raw material-conditioning material feeding pathway 91 for feeding the secondary fermented product (raw material-conditioning material) from the secondary fermentative treatment facility 80 to the raw material supply means 20 via the second distribution and supply device 90.

The second distribution and supply device 90 is connected to a carbonized raw material feeding pathway 92 for feeding the secondary fermented product to a carbonization facility 100. The carbonization facility 100 is configured to carbonize, preferably activate and carbonize, the fed secondary fermented product to produce a carbide, preferably activated carbon. The carbonization facility itself may have a conventional structure or configuration, and thus detailed description thereof will be omitted. Since the carbide, preferably activated carbon, produced in the carbonization facility 100, has a water content as low as about 10% (also in view of its high porosity), it can be used by itself as a water content adjuster for the raw material as well as the secondary fermented product itself. However, it will be described here as a raw material for forming a mixture with the secondary fermented product.

A first mixture raw material feeding pathway 93 for feeding the secondary fermented product is connected between the second distribution and supply device 90 and mixing means 110, and a second mixture raw material feeding pathway 94 for feeding the carbide is connected between the carbonization facility 100 and the mixing means 110. The mixing means 110 is configured to sufficiently mix the fed secondary fermented product and carbide, using rotating agitation blades or the like to form a mixture. In this process, it is desirable to change the ratio between the secondary fermented product and the carbide, depending on the water content of the primary fermented product, etc. This ratio is changed by adjusting the amount of secondary fermented product from the second distribution and supply device 90 or by providing supply amount control means in the carbonization facility 100 to adjust the amount of carbide to be supplied to the mixing means 110. During this mixing, the secondary fermented product itself or the microorganism (or its spores) in the secondary fermented product is adsorbed on the pores of the carbide, and the carbide becomes at least a carrier of the microorganism (or its spores). The second distribution and supply device 90 is connected to a second useful product raw material supply pathway 95 for supplying the secondary fermented product to second useful resource processing means (not shown).

The mixing means 110 is connected to a third distribution and supply device 120 comprising a valve mechanism as a four-way valve, similar to the second distribution and supply device 90. This third distribution and supply device 120 is configured to distributively supply at least part of the mixture to the raw material supply means 20, the after-mentioned microorganism activation device 130, and third useful resource processing means (not shown) to process the mixture into a third useful product of a third form.

For the purpose of the above, a first mixture feeding pathway 96 is connected between the third distribution and supply device 120 and the raw material supply means 20, and a second mixture feeding pathway 97 is connected between the third distribution and supply device 120 and microorganism activation device 130. Further, a third mixture feeding pathway 98 is connected between the third distribution and supply device 120 and third useful resource processing means.

Here, both the secondary fermented product and the mixture are supplied to the raw material supply means 20. Their switching and mixing state can be controlled based on the state of the primary fermented product determined by a measurement value of the water content of the primary fermented product from the water content sensor 44.

The microorganism activation device 130 is configured to receive the mixture from the mixing means 110, and further receive oxygen and water, respectively, from an oxygen supply means (not shown) and water supply means (not shown), and to maintain the temperature of the received water at 10°C to 40°C and the oxygen concentration at 1 to 10 mg/L to germinate and activate the spores in the mixture to produce an activated microorganism-containing mixture. The microorganism activation device 130 is connected to a biological treatment tank 210 of a sewage treatment facility 200 via an activated microorganism-containing mixture feeding pathway 220, and configured to receive a supply of the activated microorganism-containing mixture via the pathway 220. In the biological treatment tank 210, the activated microorganism in the activated microorganism-containing mixture contributes to biological treatment of sewage, and the carbide (particularly, activated carbon) in the activated microorganism-containing mixture contributes to significant reductions in the BOD and COD of the sewage. For example, a dehydrator 15 as the dehydration means is configured to receive and dehydrate returned sludge from a final sedimentation tank 212 connected to the biological treatment tank 210 of the sewage treatment facility. For this purpose, a return sludge feeding pathway 230 is connected between the dehydrator 15 and the final sedimentation tank 212.

Here, the second useful product means a further dried powdery fertilizer (compost), etc., because the water content of the secondary fermented product used as raw material therefor is set to be less than that of the primary fermented product. Further, the third useful product means a material which performs the action of fertilizer (comport) of the secondary fermented product, and an action/function of ameliorant from a carbon fixation function of the carbide.

Here, the operations of the second and third distribution and supply devices 90, 120 during the normal state (in which the water content indicated by the signal S1 is equal to or less than the given value) and during the non-normal state (in which the water content indicated by the signal S1 is greater than the given value) will be described. First of all, the operations of the second distribution and supply device 90 will be described as an example.

First, as preconditions, assume that dehydrated sludge (raw material) m with a water content of 85% is discharged from the dehydrator 15 in an amount of 200 kg per hour, for 5 hours per day. Thus, the raw material is supplied to the raw material supply means 20 in an amount of 1000 kg per day.

### Normal State

Assuming that with respect to 100 parts by mass of the raw material (dehydrated sludge), 5 parts by mass of the mixture (raw material-conditioning material (secondary fermented product: carbide = 1:1, thus water content: 20%) is supplied to the raw material supply means 20, the mixture supplied from the distribution and supply device 102 to the raw material supply means 20 is 10 kg per hour, and for 5 hours per day in synchronization with the dehydrator 15. Thus, the mixture is supplied in an amount of 50kg per day.

Therefore, the discharge amount of the mixture from the mixing means 110 needs to be 50 kg or more per day, because the mixture is supplied in an amount of 10 kg or more per hour, and for 5 hours per day in synchronization with the dehydrator 15. That is, the total amount of the mixture discharged from the mixing means 110 is the sum of a required amount for the raw material-conditioning material and a surplus amount.

The surplus amount is supplied via a supply passage 98 to third useful resource processing means (not shown) to process the mixture into a third useful product as a third useful resource.

The setting of a total amount based on the required amount and the surplus amount, the setting of a total discharge amount per hour for the synchronization, and instructions for opening/closing of the three discharge ports of the four-way valve to determine distribution amounts, are performed by the control panel.

Thus, when the total discharge amount per hour is instructed to an operational control means of the mixing means by the operation panel, this operational control means operates to cause discharge means of the mixing means to discharge the mixture in the instructed discharge amount per hour.

On the other hand, when the distribution amounts (distribution ratio) are instructed by the operation panel, operational control means to control the operation of the four-way valve operates to distribute the mixture supplied from the discharge means of the mixing means in the discharge amounts per hour, in the instructed distribution amounts, and supply the distributed mixtures to the raw material supply means 20 and the third useful resource processing means, respectively.

The above will be described using specific numerical values. The following description will be made on the assumption that the secondary fermented product is supplied to the raw material supply means 20 in an amount of 10 kg per hour, and to the second useful resource processing means in an amount of 5 kg per hour. That is, the ratio of the supply amount to the raw material supply means 20 supplied via the four-way valve 104 : the supply amount to the second useful resource processing means via the four-way valve 104 is 2 : 1.

Thus, during the normal state, the mixture is discharged from the discharge means of the mixing means in an amount of 15 kg per hour, and is distributed by the ratio of 2:1 via the four-way valve, whereby "2/3" of the discharged mixture is supplied to the raw material supply means 20, and "1/3" of the discharged mixture is supplied to the second useful resource processing means.

### Non-normal State

During the non-normal state (state in which the water content of the primary fermented product is greater than the given value (35%)), the supply amount of the mixture to the raw material supply means 20 is increased as compared to that during the normal state. Here, consider that the supply amount is increased by five times, i.e., to 50 kg per hour. When the supply amount of the mixture to the third useful resource processing means is set to 0 during the non-normal state, the discharge port of the four-way valve to the third useful resource processing means is closed, and the supply amount to the raw material supply means 20 is set to 100%. Theus, since the mixture is supplied to the raw material supply means 20 in an amount of 50 kg per hour, for 5 hours per day, i.e., in a total amount of 250 kg per day.

Consequently, during the non-normal state, the amount per day of the raw material m and the amount per day of the secondary fermented product (raw material-conditioning material) are, respectively, 1000 kg and 250 kg. Further, the water content of the raw material supplied by the conveyor 24 is 74%, i.e., is reduced by 8% as compared to that during the normal state, and the solid content is increased by more than 2.3 times as compared to that during the normal state, so that the amount of the microorganism contributing to fermentation is also increased (because the microorganism is carried by the secondary fermented product in the solids content, and thus the amount thereof is almost proportional to the amount of the solids content).

During the non-normal state, the supply amount of the mixture (raw material-conditioning material) to the material supply means 20 is increased as compared to that during the normal state, in the above manner, so that the water content of the raw material to be supplied to the primary fermentative treatment device 11 is reduced, and the amount of the microorganism contributing to fermentation to promote fermentation is increased as comparted to that during the normal state, thereby gradually returning the state of fermentation (indicated by the water content value) of the primary fermented product discharged from the primary fermentative treatment device 11 to a normal state (normal value: 35% or less).

The increase in the supply amount of the mixture (raw material-conditioning material) to the raw material supply means 20 is preferably set to 2 times to 10 times when said supply amount during the normal state is defined as 1. The upper limit of this increase is determined by taking into account the margin of the fermentative treatment container 12 of the primary fermentative treatment device 11 during the normal state (the amount of the raw material input to the fermentative treatment container 12 is 70 to 80% of an internal volume of the fermentative treatment container 12, i.e., there is a space of 30 to 20% in the fermentative treatment container 12, during the normal state).

The storage part 84 is provided with an upper limit sensor and a lower limit sensor to monitor whether the amount of the secondary fermented product stored therein is appropriate (the appropriateness is determined by the height of the top surface of the secondary fermented product).

The upper limit sensor is connected to an operational control means for controlling discharge means of the storage part 84 and the operational control means 38 of the three-way valve 36, and is configured to send a signal S3 to each of the two operational control means when the amount of the secondary fermented product stored in the storage part 84 exceeds a given upper limit (when the top surface of the stored secondary fermented product exceeds an upper limit level).

Upon receiving the signal S3, the operational control means operates to activate discharge means to discharge the stored secondary fermented product from the storage part 84. Further, upon receiving the signal S3, the operational control means operates to set the four-way valve such that the secondary fermented product is distributed only to the supply passage, and at this time, supply the secondary fermented product only to the second useful resource processing means.

The lower limit sensor is connected to operational control means of the three-way valve and is configured to send a signal S4 to this operational control means when the amount of the secondary fermented product stored in the storage part 84 falls below a given limit (when the top surface of the stored secondary fermented product becomes lower than a lower limit level). Upon receiving the signal S4, the operational control means operates to set the three-way valve such that the amount of the primary fermented product supplied to the first feed passage 40 is increased, thereby increasing the amount of the secondary fermented product stored in the storage part 84.

In the above, the raw material-conditioning material, which is primarily a material for adjusting the water content of the raw material in the raw material supply means 20, was described as a mixture of the secondary fermented product and the carbide. However, the raw material-conditioning material may be composed of only the secondary fermented product.

When the mixture is used as the raw material-conditioning material as described above, it is also important to use only the secondary fermented product as the raw material conditioning material, as the need arises, to adjust the amount of microorganism. The same can also be said about a case where the mixture is used for the biological treatment tank for sewage treatment.

Heating of the raw material to be supplied to the primary fermentative treatment device 11 is also effective in promoting the above-mentioned fermentation. An example of this will be described below with reference to FIG. 2. The sewage sludge-to-resource recycling system illustrated in FIG. 2 will be described by placing importance on a mechanism for the above heating. Thus, in FIG. 2, illustration of the secondary fermentative treatment facility and elements or components related only thereto is omitted, and their descriptions in the DESCRIPTION is also omitted.

A jacket 150 is disposed around the raw material hopper 22 to surround the raw material hopper with a gap relative to an outer wall of the raw material hopper, so that a heating gas space 152 for allowing a heating gas to pass therethrough to heat organic waste as the raw material within the raw material hopper.

A heating gas passage 160 is provided to extend from the discharge end 12b of the fermentative treatment container 12 to the heating gas space 152 to supply, as the heating gas, exhaust gas generated due to fermentation within the fermentative treatment container, from the fermentative treatment container to the heating air space. An exhaust fan 162 is interposed in the heating gas passage 160 at a position on the side of the other end 12b of the fermentative treatment container 12, to discharge the discharge gas generated due to fermentation within the fermentative treatment container 12, outside the primary fermentative treatment device.

The heating gas passage 160 is provided with heating means 170 for heating the discharge gas passing therethrough. As mentioned above, in the sewage sludge-to-resource recycling system according to the present invention, hyperthermophiles are used for fermentation of organic waste. Thus, the heating means 170 is provided to heat the heating gas for the purpose of promoting fermentation of dehydrated sludge as the raw material, and further reducing the water content of the dewatered sludge.

A heating means control means 172 is provided to control the operation of the heating means 170. In order to control the heating means 170 according to the above purpose by the heating means control means 172, the heating gas passage 160 is provided with discharge gas temperature monitoring means 174 to detect and monitor the temperature of the heating gas being flowing therethrough. Further, raw material temperature monitoring means 176 is provided in the raw material hopper 22 to detect and monitor the temperature of the raw material within the raw material hopper.

The water content sensor 44 provided within the fermentative treatment container 12 is also connected to the heating means control means 172, and the signal S 1 issued during the non-normal state in which the water content of the primary fermented product is greater than the given value (35%) is also sent to the heating means control means 172.

Upon receiving the signal S 1, the heating means control means 172 operates to activate the heating means 170 to heat the discharge gas (heating gas) being flowing through the heating gas passage 160 to 40°C or more, and supply the heated discharge gas to the heating gas space 152.

In the above way, during the non-normal state, the dehydrated sludge as the raw material is supplied with the raw material-conditioning material (secondary fermented product) in an increased amount, so that the water content thereof is reduced, and the amount of microorganism used for fermentation is increased, and further the dehydrated sludge is subjected to the heating, so that the activity of the microorganism is enhanced, whereby fermentation is promoted, and the fermentation state (indicated by the water content value) of the primary fermented product can be returned to the normal state (normal value: 35% or less) at higher speed.

Preferably, a heating gas introduction conduit 54 is connected to the heating gas space 152 to introduce the heating gas from the heating gas space 152 into an upper space of the fermentative treatment container 12. This heating gas introduction conduit 154 allows the heating gas to be introduced into the upper space of the fermentative treatment container 12 to promote fermentation by heating.

A temperature sensor 156 may be provided in the raw material hopper 22 to monitor the temperature of the supplied raw material. This temperature sensor 156 is configured to, when the temperature of the raw material is equal to or less than a given value, sect a signal to the control means 172, irrespective of the water content of the primary fermented product, to activate the heating means 170 to increase the temperature of the heating gas to increase the temperature of the raw material. This heating can contribute to preventing the temperature of dehydrated sludge from extremely dropping in the wintertime or the like, leading to a situation where the fermentation of the raw material is not complete and delayed, and the primary fermented product reaches the non-normal state.

In the sewage sludge-to-resource recycling system 10 according to the present invention configured as above, during the non-normal state, the raw material, which is dewatered sludge, is supplied with the raw material-conditioning material (mixture of the secondary fermented product and the carbide) in an increased amount, so that the water content thereof is reduced, and the amount of microorganism used for fermentation is increased, whereby fermentation is promoted, and the non-normal state can be quickly returned to the normal state.

### LIST OF REFERENCE SIGNS

10: sewage sludge-to-resource recycling system
11: primary fermentative treatment device
12: fermentative treatment container
15: dehydrator
20: raw material supply means
22: raw material hopper
24: conveyer
30: discharge means
34: first distribution and supply device
80: secondary fermentative treatment facility
82: production part 82
84: storage part
90: second distribution and supply device
100: carbonization facility
110: mixing means 110
120: third distribution and supply device
130: microorganism activation device
200: sewage treatment facility
210: biological treatment tank
212: final sedimentation tank

## Claims

1. A sewage sludge-to-resource recycling system of circulation-type for recycling sewage sludge as organic waste into a useful product, the sewage sludge-to-resource recycling system of circulation-type comprising:
dehydration means to dehydrate sewage sludge until a water content of the dehydrated sewage sludge becomes a preset given raw material water content value, to form a raw material;
primary fermentative treatment means comprising a fermentative treatment container which is formed in a horizontal cylindrical shape and configured to be rotated about a central axis thereof, the primary fermentative treatment means being configured to rotate the fermentative treatment container about the central axis to cause the raw material within the fermentative treatment container to gradually move from an inlet end side toward a discharge end side of the fermentative treatment container over a given number of days, thereby fermentatively treating the raw material by an aerobic microorganism to produce a primary fermented product having a first set water content value or less;
raw material supply means provided on the inlet end side of the fermentative treatment container and configured to receive the raw material from the dehydration means and supply the received raw material into the fermentative treatment container;
primary fermented product discharge means provided on the discharge end side of the fermentative treatment container and configured to discharge the primary fermented product outside the fermentative treatment container:
secondary fermentative treatment means to receive the primary fermented product from the primary fermentative treatment means, and additionally ferment the received primary fermented product to produce a secondary fermented product having a second set water content value or less, the second set water content value being less than a water content of the primary fermented product;
carbonization means to receive the secondary fermented product from the secondary fermentative treatment means, and subject the received secondary fermented product to carbonization treatment to produce a carbide;
mixing means to receive the secondary fermented product from the secondary fermentative treatment means and the carbide from the carbonization means, and mix them together to produce a mixture; and
water content measurement means to measure the water content of the primary fermented product discharged from the primary fermentative treatment means;
wherein:
the primary fermented product discharge means is connected to first distribution and supply means, the first distribution and supply means being configured to, in a normal state in which a water content value of the primary fermented product measured by the water content measurement means is equal to or less than the first set water content value, distribute a first given amount of the primary fermented product and a remaining amount of the primary fermented product, respectively, to the secondary fermentative treatment means, and first useful resource processing means to perform processing into a first useful product of a first form, and in a non-normal state in which said water content value exceeds the first set water content value, supply an entire amount of the primary fermented product to the secondary fermentative treatment means;
the secondary fermented product discharge means is connected to second distribution and supply means, the second distribution and supply means being configured to distributively supply at least part of the secondary fermented product to the carbonization means and the mixing means; and
the mixing means is connected to third distribute and supply means, the third distribution and supply means being configured to distributively supply at least part of the mixture to the raw material supply means and/or third useful resource processing means to perform processing into a third useful product of a third form.

2. The sewage sludge-to-resource recycling system as defined in claim 1, wherein
the aerobic microorganism is gram-positive aerobic thermophiles, and
the secondary fermentative treatment means is the heaping-type fermentative treatment means, and comprises: a production part to heap the primary fermented product supplied from the first distribution and supply means, and fermentatively treat the heaped primary fermented product by the gram-positive aerobic thermophiles to produce the secondary fermented product such that it has the second set water content value less than the first set water content value for the primary fermented product; a storage part to store the secondary fermented product produced in the production part; and secondary fermented product discharge means to discharge the secondary fermented product from the storage part by a given amount.

3. The sewage sludge-to-resource recycling system as defined in claim 1, wherein the third distribute and supply means is configured to, in the non-normal state, distributively supply the mixture to the raw material supply means.

4. The sewage sludge-to-resource recycling system as defined in claim 1, wherein the third distribute and supply means is configured to supply a given amount of the mixture to a biological treatment part of a sewage treatment facility via microorganism activation means, in a state in which the aerobic microorganism in the secondary fermented product of the mixture is activated.

5. The sewage sludge-to-resource recycling system as defined in claim 2, wherein the secondary fermentative treatment means comprises a supply conveyer for transferring the primary fermented product supplied from the first distribution and supply means, to the production part.

6. The sewage sludge-to-resource recycling system as defined in claim 2, wherein the secondary fermentative treatment means comprises transfer means to transfer the secondary fermented product produced in the production part, from the production part to the storage part.

7. The sewage sludge-to-resource recycling system as defined in claim 2, wherein the production part of the secondary fermentative treatment means comprises:
second water content measurement means to measure a water content value of the produced secondary fermented product;
feeding and returning means provided within the production part over an approximately entire length of the production part and configured to be switchable between a feeding state in which the primary fermented product is fed from an upstream side to a downstream side of the production part, and a returning state in which the secondary fermented product is returned from the downstream side to the upstream side; and
operational control means to control an operational state of the feeding and returning means such that it is switched between the feeding state and the return state, depending on the water content value of the secondary fermented product measured by the second water content measurement means.

8. The sewage sludge-to-resource recycling system as defined in claim 7, wherein the operational control means is configured to control the operational state of the feeding and returning means such that it is switched to the return state when the water content value of the secondary fermented product measured by the second water content measurement means exceeds the second set water content value.

9. The sewage sludge-to-resource recycling system as defined in claim 1, wherein the first set water content value is a value of 35% or less, and the second set water content value is set to be less than the first set water content value by 2 to 15%.

10. The sewage sludge-to-resource recycling system as defined in claim 1, wherein the mixture after being processed by the third useful resource processing means is a material usable as both ameliorant and compost/fertilizer.

11. The sewage sludge-to-resource recycling system as defined in claim 1, wherein the fermentative treatment container is provided with agitation means to agitate the raw material thereinside.

12. The sewage sludge-to-resource recycling system as defined in claim 11, wherein the agitation means is rotation actuation means to rotate the fermentative treatment container.

13. The sewage sludge-to-resource recycling system as defined in claim 1, wherein the raw material supply means comprises:
a raw material hopper configured to temporarily store the raw material from the dehydration means;
a jacket surrounding the raw material hopper with a gap relative to an outer wall of the raw material hopper, to form a heating gas space for allowing a heating gas to pass therethrough to heat the raw material within the raw material hopper; and
a heating gas passage extending from the discharge end side of the fermentative treatment container to the heating gas space, the heating gas passage being configured to supply, as the heating gas, a discharge gas generated due to fermentation within the fermentative treatment container, from the fermentative treatment container to the heating gas space.

14. The sewage sludge-to-resource recycling system as defined in claim 1, wherein the aerobic microorganism includes microorganisms belonging to gram-positive Bacilli of Firmicutes, and/or microorganisms belonging to gram-positive Actinobacteria of Actinomycetota, and/or gram-positive bacteria of Chloroflexi.

15. The sewage sludge-to-resource recycling system as defined in claim 1, wherein the second distribution and supply means is configured to distributively supply at least part of the secondary fermented product to the raw material supply means and/or second useful resource processing means to perform processing into a second useful product of a second form.
